(19) Europäisches Patentamt
European Patent Office
Office européen des brevets

(11) Veröffentlichungsnummer: **0 322 537 A2**

(12) # EUROPÄISCHE PATENTANMELDUNG

(21) Anmeldenummer: 88117877.6

(22) Anmeldetag: 27.10.88

(51) Int. Cl.4: C07C 45/58 , C07C 49/385 , C07C 49/413 , C07C 49/587 , C07C 49/607

(30) Priorität: 24.12.87 DE 3744094

(43) Veröffentlichungstag der Anmeldung: 05.07.89 Patentblatt 89/27

(84) Benannte Vertragsstaaten: BE CH DE ES FR GB IT LI NL

(71) Anmelder: HÜLS AKTIENGESELLSCHAFT
Patentabteilung / PB 15 - Postfach 13 20
D-4370 Marl 1(DE)

(72) Erfinder: Büschken, Wilfried, Dr.
Rosenkamp 10
D-4358 Haltern 6(DE)

(54) **Verfahren zur Herstellung von cyclischen Ketonen durch Isomerisierung von Epoxiden.**

(57) Es war Aufgabe der Erfindung, ausgehend von Epoxiden, ein gegenüber dem Stand der Technik wenig aufwendiges, nur kurze Reaktionszeiten benötigendes und in bezug auf Peroxidbildung risikoloses Verfahren zu finden. Gemäß dem Stand der Technik wird insbesondere mit Diethylether als Lösungsmittel und Magnesiumverbindungen als Katalysator gearbeitet.

Die Lösung des Problems bestand in dem Einsatz polarer Lösungsmittel, wie z. B. N,N′-disubstituierten cyclischen Harnstoffen, in Gegenwart von Alkali- oder Erdalkalihalogeniden.

Die cyclischen Ketone insbesondere mit 15 bis 17 Kohlenstoffatomen werden als Riechstoffe eingesetzt.

EP 0 322 537 A2

# Verfahren zur Herstellung von cyclischen Ketonen durch Isomerisierung von Epoxiden

Die Erfindung betrifft ein Verfahren zur Herstellung von cyclischen Ketonen mit 7 bis 20 Ringkohlenstoffatomen, mit 0 bis 5 Mehrfachbindungen und mit einer Seitenkette, die aus 0 bis 5 Kohlenstoffatomen besteht, aus den entsprechenden Epoxiden (Oxiranen), die technisch leicht aus cyclischen Olefinen erhalten werden können.

Beispielsweise handelt es sich um die Herstellung folgender Verbindungen:

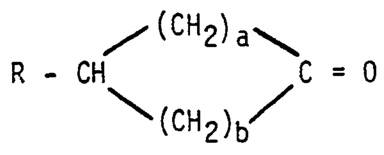

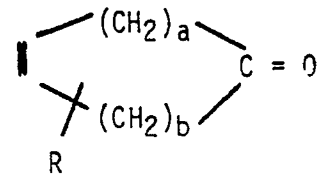

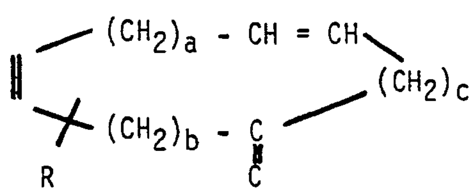

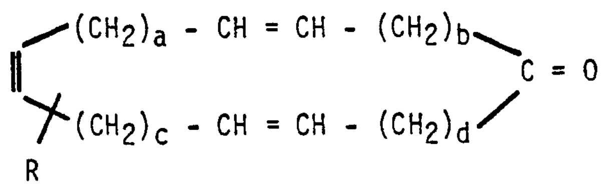

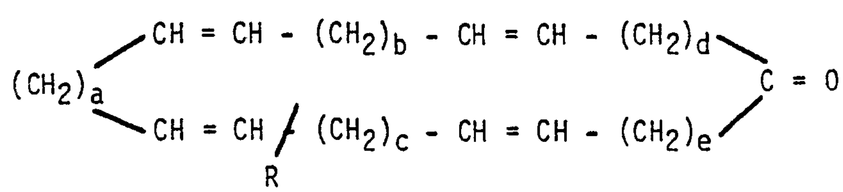

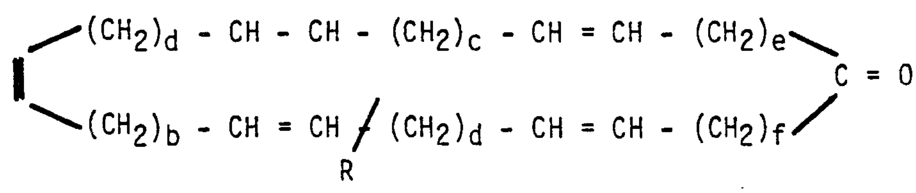

wobei hier a, b, c, d, e und f in jeder Verbindung so gewählt ist, daß die Zahl der Ringkohlenstoffatome zusammen 7 bis 20 beträgt und

R = H; $CH_3$; $C_2H_5$; $C_2H_3$; $C_3H_7$; $C_3H_5$; $C_4H_9$; $C_4H_7$; $C_5H_{11}$ oder $C_5H_9$

bedeuten kann.

Diese Ketone sind z. B. Zwischenstufen für die Herstellung von Lactamen, Aminen, Carbonsäuren und einer Vielzahl von weiteren Verbindungen. Cyclische Ketone mit 15 bis 17 Kohlenstoffatomen sind wertvolle Riechstoffe.

Gemäß DE-PS 10 75 601 wird die Isomerisierung von Epoxiden zu den entsprechenden cyclischen Ketonen mit wasserfreien Halogeniden von Elementen aus den Gruppen IIA und IIIA des Periodensystems (Bezeichnung entsprechend Chemical Abstracts), insbesondere mit Magnesiumjodid und Magnesiumbromid in Diethylether durchgeführt. Nach Entfernung der Salze durch Wasserwäsche werden die Ketone durch Destillation gewonnen.

Dieses Verfahren weist folgende Nachteile auf:

a) je nach Substrat liegen die Reaktionszeiten für einen Umsatz größer als 95 % bei 15 bis 70 h.

b) die Verwendung von Diethylether ist wegen seines niedrigen Flammpunktes, seiner hohen Flüchtigkeit und seiner Neigung zur Bildung von Peroxiden riskant.

c) die verwendeten Katalysatoren werden nicht zurückgewonnen und belasten das Abwasser.

Es bestand daher die Aufgabe, ein Verfahren zur Herstellung von cyclischen Ketonen zu finden, das technisch wenig aufwendig und ohne Risiko durchführbar ist und nicht die oben genannten Nachteile aufweist.

Überraschenderweise wurde gefunden, daß Epoxide im Temperaturbereich von 120 bis 250 °C, insbesondere von 150 bis 200 °C, in einem polaren Lösungsmittel in Gegenwart von Alkali- oder Erdalkalihalogeniden in sehr guten Ausbeuten zu den entsprechenden Ketonen umgelagert werden, und daß die Aufarbeitung und Zurückgewinnung des Lösungsmittel und Katalysators in technisch leicht durchführbaren Verfahrensschritten möglich ist.

Gegenstand der Erfindung ist daher ein Verfahren zur Herstellung von cyclischen Ketonen mit 7 bis 20 Ringkohlenstoffatomen mit 0 bis 5 Mehrfachbindungen und mit einer Seitenkette mit 0 bis 5 Kohlenstoffatomen, insbesondere Ketonen mit 15 bis 17 Kohlenstoffatomen, aus den entsprechenden Epoxiden, welches dadurch gekennzeichnet ist, daß die Umlagerung mit Alkali- und/oder Erdalkalihalogeniden in einem polaren Lösungsmittel bei Temperaturen von 120 bis 250 °C durchgeführt wird.

Es war nicht vorhersehbar, daß beim erfindungsgemäßen Verfahren trotz der relativ hohen Temperatur die Konkurrenzreaktionen, wie Aldehydbildung unter Ringverengung, Bildung von cyclischen Allylalkoholderivaten, transannulare Wasserstoffverschiebungen und bei olefinischen Epoxiden die Bildung von Bicyclen vernachlässigbar gering sind.

Als Lösungsmittel werden polare, aprotische Lösungsmittel, die mit dem Salz und Substrat eine homogene Lösung bilden können und mit den Epoxiden und den daraus entstehenden Ketonen keinerlei Reaktion eingehen, wie z. B. N,N′-disubstituierte cyclische Harnstoffe, N-substituierte Lactame und N,N-disubstituierte Säureamide, verwendet. Die Lösungsmittelmenge soll dabei 20 bis 300 Gew.-%, insbesondere 80 bis 120 Gew.-%, bezogen auf die Epoxidmenge, betragen. Als Katalysatoren sind Alkali- und Erdalkalihalogenide, wie z. B. Magnesiumjodid und Magnesiumbromid, insbesondere aber Natriumjodid und Lithiumchlorid, geeignet. Für die vollständige Umsetzung sind in der Regel nicht mehr als 7 h notwendig. Die Salze werden dabei in Mengen von 0,5 bis 20 Gew.-%, insbesondere 2 bis 7 Gew.-%, bezogen auf die Epoxidmenge, eingesetzt.

Die Aufarbeitung ist wenig aufwendig. Das Lösungsmittel wird abdestilliert und der Katalysator abfiltriert. Das Filtrat wird mit Wasser gewaschen und rektifiziert. Katalysator und Lösungsmittel werden nahezu quantitativ zurückgewonnen.

Gegenüber den bekannten Verfahren hat das erfindungsgemäße Verfahren folgende Vorteile:

a) höhere Substratkonzentration und kürzere Reaktionszeiten, woraus sich eine wesentlich bessere Raum-Zeit-Ausbeute ergibt

b) Verwendung eines technisch unproblematischen Lösungsmittels

c) Zurückgewinnung des Lösungsmittels und des eingesetzten Katalysators und somit nahezu keine Salzfracht im Abwasser.

Beispiel 1

25 g Epoxicyclododecan und 1 g Lithiumchlorid werden in 20 g N,N′-Dimethylethylenharnstoff gelöst und die Lösung 6 h bei 200 °C gerührt. Im Reaktionsgemisch wurde Cyclododecanon in 94 %iger Ausbeute nachgewiesen.

Beispiel 2

Eine Lösung aus 25 g Epoxicyclododecan, 1 g Lithiumchlorid und 20 g N-Methylpyrrolidon(2) wurde 6 h bei 200 °C gerührt. Im Reaktionsgemisch war Cyclododecanon in 93,5 %iger Ausbeute enthalten.

Beispiel 3

Eine Lösung aus 12,5 g 1,2-Epoxicyclododeca-5,9-dien, 10 g N,N′-Dimethylethylenharnstoff und 0,5 g Natriumjodid wurde 6 h bei 200 °C gerührt. Die gaschromatographisch ermittelte Ausbeute betrug 95 %.

Beispiel 4

Eine Lösung aus 25 g 1,2-Epoxicyclododeca-5,9-dien, 20 g N,N'-Dimethylethylenharnstoff und 1 g Lithiumchlorid wurden 6 h bei 200 °C gerührt. Bei einem Umsatz von 90,7 % lag die Ausbeute bei 87,3 %.

Beispiel 5

Eine Lösung aus 25 g 1,2-Epoxicyclododeca-5,9-dien, 20 g N-Methylpyrrolidon(2) und 1 g Lithiumchlorid wurde 6 h bei 200 °C gerührt. Bei einem Umsatz von 89,5 % wurde eine Ausbeute von 87,3 % erhalten.

Beispiel 6

11,8 g 1,2-Epoxicyclohexadec-9-en, 5 ml N,N'-Dimethylethylenharnstoff und 0,5 g Natriumjodid wurde 6 h bei 200 °C gerührt. Cyclohexadec-8- enon(1) entstand in 81 %iger Ausbeute.

Beispiel 7

Eine Lösung aus 500 g 97,6 %igem 1,2-Epoxicyclohexadec-9-en, 400 g N,N'-Dimethylethylenharnstoff und 20 g Lithiumchlorid wurde 7 h bei 200 °C gerührt. Über eine 20 cm lange Füllkörperkolonne wurde das Lösungsmittel abdestilliert (10 mbar; 106 °C). Es wurden 390 g N,N'- Dimethylethylenharnstoff in einer Reinheit von 96,3 % als Destillat erhalten. Das entsprach einer Zurückgewinnungsrate von 94 %. Nach dem Absaugen von Lithiumchlorid (19,72 g = 98,6 % des Einsatzes) wurde dem Filtrat 300 g Toluol zugesetzt und dreimal mit je 100 ml Wasser gewaschen. Nach dem Abziehen von Toluol und von emulgiertem Wasser blieb ein Rückstand von 498 g mit 90,5 % an Cyclohexadec-8-enon. Dies entsprach einer Ausbeute von 92,3 %.

Beispiel 8

Eine Lösung aus 97,6 %igem 1,2-Epoxicyclohexadec-9-en, 560 g N-Methylpyrrolidon(2) und 28 g Lithiumchlorid wurde 7 h bei 200 °C gerührt. Über eine 20 cm lange Füllkörperkolonne wurde das Lösungsmittel abdestilliert (p = 14 bis 16 mbar, Kopftemperatur 80 bis 85 °C; Sumpftemperatur 101 bis 160 °C). Es wurden 532 g N-Methylpyrrolidon mit einer Reinheit von 99,1 % erhalten (94 % des Einsatzes). Das ausgefallene Lithiumchlorid (~ 28 g ≅ 100 % des Einsatzes) wurde abfiltriert und mit 300 g Toluol gewaschen. Das Filtrat wurde dreimal mit je 100 ml Wasser extrahiert (Mischzeit jeweils 5 Minuten, Absitzzeit jeweils 15 Minuten). Nach Abdestillieren des Toluols und emulgiertem Wasser blieb ein Rückstand von 731 g mit 89,6 % Cyclohexadec-8-enon(1) (= 95,9 % der Theorie).

Beispiel 9

10,5 g Epoxicyclohexadecan, 10 g N,N'-Dimethylethylenharnstoff und 0,5 g Lithiumchlorid werden 6 h bei 200 °C gerührt. Bei einem Umsatz von 100 % betrug die Ausbeute an Cyclohexadecanon 98,5 %.

Beispiel 10

Ein Gemisch aus 10,5 g Epoxicyclohexadecan, 10 g N-Methylpyrrolidon und 0,5 g Lithiumchlorid wurden 6 h bei 200 °C gerührt. Bei einem Umsatz von 100 % betrug die Ausbeute an Cyclohexadecanon 98,9 %.

**Ansprüche**

1. Verfahren zur Herstellung von cyclischen Ketonen mit 7 bis 20 Ringkohlenstoffatomen mit 0 bis 5 Mehrfachbindungen und mit einer Seitenkette mit 0 bis 5 Kohlenstoffatomen, insbesondere Ketonen mit 15 bis 17 Kohlenstoffatomen, aus den entsprechenden Epoxiden,
dadurch gekennzeichnet,
daß die Umlagerung mit Alkali- und/oder Erdalkalihalogeniden in einem polaren Lösungsmittel bei Temperaturen von 120 bis 250 °C durchgeführt wird.

2. Verfahren nach Anspruch 1,
dadurch gekennzeichnet,
daß als Katalysatoren Natriumjodid und Lithiumchlorid eingesetzt werden.

3. Verfahren nach Anspruch 1 und 2,
dadurch gekennzeichnet,
daß die Salze in Mengen von 0,5 bis 20 Gew.-%, vorzugsweise 2 bis 7 Gew.-%, bezogen auf das Epoxid, eingesetzt werden.

4. Verfahren nach den Ansprüchen 1 bis 3,
dadurch gekennzeichnet,
daß als Lösungsmittel N,N-disubstituierte Dialkylcarbonsäureamide, N-substituierte Lactame, N,N,N′,N′-tetrasubstituierte Harnstoffe und N,N′-disubstituierte cyclische Harnstoffe eingesetzt werden, insbesondere N-Methylpyrrolidon(2) und N,N′-Dimethylethylenharnstoff.

5. Verfahren nach den Ansprüchen 1 bis 4,
dadurch gekennzeichnet,
daß die Lösungsmittelmenge 20 bis 300 Gew.-%, insbesondere 80 bis 120 Gew.-%, bezogen auf eingesetztes Epoxid, beträgt.

6. Verfahren nach den Ansprüchen 1 bis 5,
dadurch gekennzeichnet,
daß die Reaktion vorzugsweise bei Temperaturen von 150 bis 200 °C durchgeführt wird.